# EUROPEAN PATENT APPLICATION

(11) **EP 4 257 384 A1**
(43) Date of publication of application: **11.10.2023**
(21) Application number: 22742464.5
(22) Date of filing: 12.01.2022
(51) Int. Cl.: B60H 3/00, A61L 9/20

(54) **LIGHT ILLUMINATION UNIT AND VEHICLE AIR-CONDITIONING DEVICE**

(30) Priority: 25.01.2021 JP 2021009669
(71) Applicant: Mitsubishi Heavy Industries Thermal Systems, Ltd., Tokyo, 100-8332 (JP)
(72) Inventor: CHIKAGAWA Noriyuki, Tokyo 100-8332 (JP); YAMAMOTO Takahide, Tokyo 100-8332 (JP); YONEDA Isami, Tokyo 100-8332 (JP)
(74) Representative: Cabinet Beau de Loménie
(86) International application number: PCT/JP2022/000636
(87) International publication number: WO 2022/158342

(57) **Abstract**

This light illumination unit comprises: a heat sink that has a plate-shaped heat sink body and fins protruding from a first main surface of the heat sink body; an electronic substrate that is laminated on a second main surface of the heat sink body on the reverse side thereof from the first main surface; and a light illumination element that is mounted on a first surface of the electronic substrate on the side thereof where the heat sink is provided. As a result of said configuration, it is possible to save space as well as power.

## Description

### Technical Field

The present disclosure relates to a light irradiation unit and a vehicle air-conditioning device.

This application claims the priority of Japanese Patent Application No. 2021-009669 filed in Japan on January 25, 2021, the content of which is incorporated herein by reference.

### Background Art

A vehicle air-conditioning device mainly includes a fan, a duct, and an evaporator. The fan is driven to supply air to the evaporator through the duct. The evaporator exchanges heat between air and a refrigerant. In this manner, the temperature-controlled air blowing can be generated.

In the related art, an example has been proposed in which a sterilizing device is provided inside the duct to provide a sterilizing effect of mildewproofing for the fan, the evaporator, and the air blowing itself. For example, PTL 1 below discloses a configuration in which a sterilizing lamp is provided inside the duct.

### Citation List

### Patent Literature

[PTL 1] Japanese Unexamined Utility Model Application, First Publication No. S63-72217

### Summary of Invention

### Technical Problem

However, when a fluorescent tube serving as the sterilizing lamp is provided inside the duct as described above, an installation space is enlarged, thereby causing a possibility that a pressure loss inside the duct may increase. In addition, there is a possibility that heat generated by the sterilizing lamp may affect a stable operation of other devices.

The present disclosure is made to solve the above-described problems, and an object of the present disclosure is to provide a light irradiation unit that can be installed in a space-saving manner and minimizes power consumption, and a vehicle air-conditioning device.

### Solution to Problem

According to the present disclosure, in order to solve the above-described problems, there is provided a light irradiation unit including a heat sink having a plate-shaped heat sink body and a fin protruding from a first main surface of the heat sink body, an electronic substrate laminated on a second main surface on a side opposite to the first main surface in the heat sink body, and a light irradiation element mounted on a first surface of the electronic substrate, which is a surface on a side where the heat sink is provided.

### Advantageous Effects of Invention

According to the present disclosure, it is possible to provide a light irradiation unit that can be installed in a space-saving manner and that minimizes power consumption, and a vehicle air-conditioning device.

### Brief Description of Drawings

Fig. 1 is a sectional view illustrating a configuration of a vehicle air-conditioning device according to an embodiment of the present disclosure.
Fig. 2 is a sectional view illustrating a configuration of a light irradiation unit according to the embodiment of the present disclosure.
Fig. 3 is a front view illustrating a configuration of a light irradiation element according to the embodiment of the present disclosure.
Fig. 4 is a schematic view illustrating a power element and a power supply system of the light irradiation element according to the embodiment of the present disclosure.

### Description of Embodiments

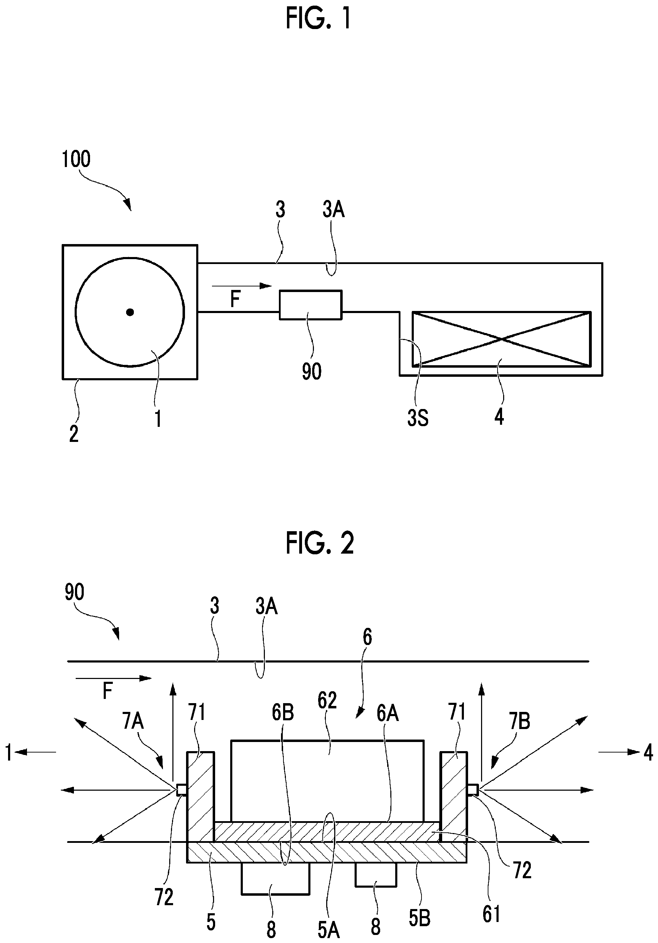

Hereinafter, a vehicle air-conditioning device 100 and a light irradiation unit 90 according to a first embodiment of the present disclosure will be described with reference to Figs. 1 to 4.

### (Configuration of Vehicle Air-Conditioning Device)

The vehicle air-conditioning device 100 is a device for performing temperature-controlled air blowing to an interior (living space) of a vehicle. As illustrated in Fig. 1, the vehicle air-conditioning device 100 includes a fan 1, a fan case 2, a duct 3, and an evaporator 4.

The fan 1 is an impeller for pumping air introduced from an outside. The fan 1 is covered from the outside by the fan case 2. The fan 1 is rotated inside the fan case 2 to blow the air of which the temperature is yet to be controlled into the duct 3 connected to the fan case 2. Driving control including switching on and off the fan 1 and rotation speed control is performed by a power element 8 (fan controller, to be described later).

A flow path through which the air pumped from the fan 1 circulates is formed inside the duct 3. The light irradiation unit 90 (to be described later) is attached to an inner wall 3A (inner surface) of the duct 3. An accommodation space 3S recessed outward from the inner wall 3A is formed in an end portion (that is, an end portion on a downstream side of the duct 3 in a first direction F of the air) on a side opposite to the fan 1 inside the duct 3. The evaporator 4 is accommodated inside the accommodation space 3S.

The evaporator 4 is a portion of a refrigerant circuit having a refrigerating cycle. The evaporator 4 exchanges heat between the air circulating inside the duct 3 and a refrigerant. In this manner, the air passing through the evaporator 4 is temperature-controlled to be cold air or warm air, and the temperature-controlled air is blown into the vehicle.

### (Configuration of Light Irradiation Unit)

The light irradiation unit 90 is provided to perform sterilization treatment to mainly achieve mildewproofing and corrosion prevention for the air blowing itself flowing inside the duct 3, the fan 1, and the evaporator 4. As illustrated in Fig. 2, the light irradiation unit 90 includes a heat sink 6, an electronic substrate 5, light irradiation elements 7A and 7B, and a power element 8.

### (Configuration of Heat Sink)

The heat sink 6 includes a heat sink body 61 and a plurality of fins 62. The heat sink body 61 has a thick plate shape spreading along the inner wall 3A of the duct 3. A portion of the heat sink body 61 is exposed to the outside of the duct 3 via an opening portion formed on the inner wall 3A of the duct 3. Out of both surfaces of the heat sink body 61 in a thickness direction, a surface on a side facing the inside of the duct 3 is a first main surface 6A. The plurality of fins 62 are provided on the first main surface 6A. The fin 62 has a plate shape protruding from the first main surface 6A in a direction orthogonal to the first main surface 6A and extending along a flowing direction of the air blowing (hereinafter, referred to as a first direction F) inside the duct 3. In addition, the fins 62 are arranged at an interval in a direction orthogonal to the first direction F.

Out of both surfaces of the heat sink body 61 in the thickness direction, a surface on a side facing the outside of the duct 3 (that is, a surface on a side opposite to the first main surface 6A) is a second main surface 6B. An electronic substrate 5 is laminated on the second main surface 6B. For example, the electronic substrate 5 is a printed substrate formed of a glass epoxy resin or Bakelite, and printed wiring is provided on at least one surface thereof. Out of both surfaces of the electronic substrate 5 in the thickness direction, a surface on a side that comes into contact with the heat sink body 61 is the first surface 5A. In the electronic substrate 5, a dimension in the first direction F is set to be larger than that of the heat sink body 61. That is, both end portions of the first surface 5A in the first direction F protrude outward from the heat sink body 61.

### (Configuration of Light Irradiation Element)

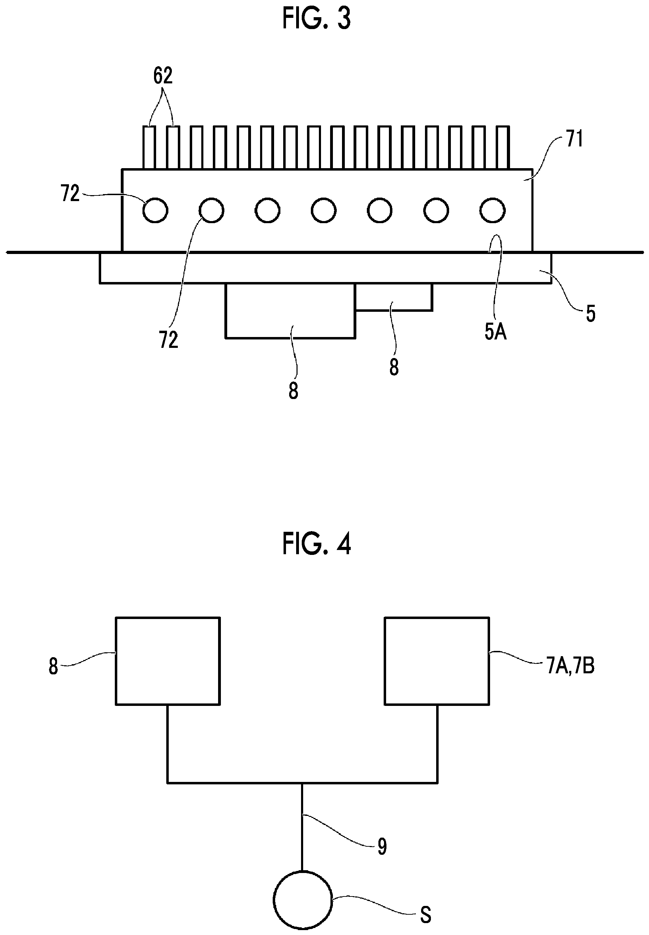

Light irradiation elements 7A and 7B are respectively provided in both end portions of the above-described first surface 5A. The light irradiation element 7A is located on the fan 1 side in the first direction F. The light irradiation element 7B is located on the evaporator 4 side in the first direction F. Each of the light irradiation elements 7A and 7B includes a plate-shaped support plate 71 protruding from the first surface 5A, and a plurality of element bodies 72 attached to a surface of the support plate 71. As illustrated in Fig. 2 or 3, a protruding height of the support plate 71 is set to be lower than a protruding height of the fins 62 of the heat sink 6. That is, when viewed in the first direction F, a tip portion of the fin 62 protrudes upward from the support plate 71. In this manner, an area blocked by the support plate 71 in the fin 62 is reduced. Therefore, heat can be more efficiently dissipated as the heat sink 6. In addition, the support plate 71 is in contact with the heat sink body 61 from both sides in the first direction F. That is, the support plate 71 and the heat sink body 61 are thermally connected. In addition, as long as both are thermally connected, a slight gap may be formed between the support plate 71 and the heat sink body 61. In this case, the support plate 71 and the heat sink body 61 are thermally connected via the electronic substrate 5.

As illustrated in Fig. 3, the plurality of element bodies 72 are arranged on a surface of the support plate 71 at an interval in a direction orthogonal to the first direction F. Specifically, an ultraviolet irradiation type LED is used as the element body 72. In particular, an LED capable of emitting an ultraviolet ray having a wavelength of 280 nm or shorter and called UVC is preferably used as the element body 72. As illustrated in Fig. 2, the element body 72 of the light irradiation element 7A performs light irradiation while a direction toward the fan 1 side is set as an optical axis center. In addition, the light irradiation is performed while a spread of approximately 60° is provided based on the optical axis center. The element body 72 of the light irradiation element 7B performs light irradiation while a direction toward the evaporator 4 side is set as the optical axis center. In addition, the light irradiation is performed while a spread of approximately 60° is provided based on the optical axis center. That is, the light irradiation elements 7A and 7B are configured to be capable of performing light irradiation in a direction intersecting with an extending direction of the fin 62. In other words, each of the light irradiation elements 7A and 7B is configured to be capable of performing light irradiation toward a side opposite to a side where the heat sink 6 is located in the first direction F. As indicated by an arrow in Fig. 2, an LED capable of performing light irradiation in the direction orthogonal to the first direction F (that is, whose spreading angle from the optical axis is approximately 90°) can be used as the element body 72.

Out of both surfaces of the electronic substrate 5 in the thickness direction, a surface on a side opposite to the first surface 5A is the second surface 5B. The power element 8 which is a portion of a control circuit for driving the fan 1 is mounted on the second surface 5B. Specifically, as the power element 8, a power transistor or a MOSFET is used. The power element 8 is driven to generate heat. The heat is transferred to the heat sink 6 via the electronic substrate 5. That is, the heat sink 6 is thermally connected to the power element 8 in addition to the above-described light irradiation elements 7A and 7B.

Furthermore, as illustrated in Fig. 4, the power element 8, the light irradiation elements 7A and 7B, and a power supply S are connected by a single harness 9. That is, when electric conduction of the harness 9 is released, power supply to the power element 8 and the light irradiation elements 7A and 7B is stopped at the same time.

### (Operational Effect)

Next, an operation of the vehicle air-conditioning device 100 and the light irradiation unit 90 according to the present embodiment will be described. In driving the vehicle air-conditioning device 100, the fan 1 is first driven. In addition, a refrigerant circuit including the evaporator 4 is operated by driving a compressor (not illustrated). In this manner, external air guided from the fan 1 through the duct 3 comes into contact with the evaporator 4, and exchanges heat with a refrigerant. As a result, temperature-controlled air blowing is supplied into the vehicle.

Here, inside the duct 3, there is a possibility that a virus causing a disease or a bacterium causing a mildew may grow and multiply. Therefore, in the present embodiment, the light irradiation unit 90 is provided inside the duct 3. A sterilization treatment can be performed on the air blowing itself circulating inside the duct 3, the fan 1 disposed in a portion connected to the duct 3, the evaporator 4, and further, the inner wall 3A of the duct 3, by using the ultraviolet ray emitted from the light irradiation unit 90. In particular, the ultraviolet irradiation type LED is used as the light irradiation elements 7A and 7B. Therefore, the sterilization treatment can be performed with less power consumption, compared to a sterilizing lamp (fluorescent tube) used in the related art.

The light irradiation elements 7A and 7B are operated to generate heat. The heat is dissipated during the air blowing circulating inside the duct 3 through the heat sink 6. Furthermore, the heat sink 6 is also used to dissipate the heat generated by the power element 8. That is, in the present embodiment, the heat sink 6 is used to cool both the power element 8 and the light irradiation elements 7A and 7B. In this manner, for example, an occupied space can be reduced, compared to a case where each dedicated heat sink for the power element 8 and the light irradiation elements 7A and 7B is provided. As a result, a dimension and a body size of the vehicle air-conditioning device 100 and the light irradiation unit 90 can be minimized.

In this way, according to the above-described configuration, the heat generated by the light irradiation elements 7A and 7B can be efficiently dissipated by the heat sink 6. Furthermore, both the fin 62 of the heat sink 6 and the light irradiation elements 7A and 7B are provided on the first surface 5A side of the electronic substrate 5. Therefore, for example, compared to a case where both of these extend in mutually different directions, the dimension and the body size of the device can be minimized. In particular, when the fin 62 protrudes outward from the duct 3, there is a possibility of interfering with other devices of the vehicle. According to the above-described configuration, the possibility can be reduced, and a device layout of the vehicle can be more freely planned.

In addition, according to the above-described configuration, the heat from the fin 62 can be dissipated by the air blowing, and the light irradiation elements 7A and 7B can be cooled. Furthermore, the light irradiation elements 7A and 7B can stably provide a sterilizing effect for the air blowing itself and other devices (fan 1, evaporator 4, and inner wall 3A of the duct 3) exposed inside the duct 3. In addition, both the light irradiation elements 7A and 7B and the heat sink 6 are provided on the same side surface of the electronic substrate 5. Therefore, the light irradiation unit 90 itself can be cooled by using a fluid itself which is a sterilizing (light irradiation) target.

Furthermore, according to the above-described configuration, power is supplied to the power element 8 and the light irradiation elements 7A and 7B from the single harness 9. Therefore, for example, during maintenance work, the power element 8 and the light irradiation elements 7A and 7B can be brought into a turned-off state at the same time by detaching (releasing electric conduction) the harness 9. In this manner, a possibility that a worker may be exposed to harmful light can be reduced. In particular, since the ultraviolet ray is invisible light, there is a high possibility that the worker may be exposed without noticing light emission during work. According to the above-described configuration, the possibility can be reduced, and the work can be more safely carried out.

Hitherto, the embodiment of the present disclosure has been described. The above-described configurations can be changed or modified in various ways as long as the change or the modification does not depart from the concept of the present disclosure. For example, in the above-described embodiment, an example has been described in which the LED emitting the UVC is used as the light irradiation elements 7A and 7B. However, the LED emitting an ultraviolet ray having other wavelengths can also be used as the light irradiation elements 7A and 7B. In addition, as another aspect of the heat sink 6, a type having a plurality of pins can also be used instead of the fin 62.

### <Additional Notes>

The light irradiation unit 90 and the vehicle air-conditioning device 100 described in each embodiment are grasped as follows, for example.

(1) The light irradiation unit 90 according to a first aspect includes the heat sink 6 having the plate-shaped heat sink body 61 and the fin 62 protruding from the first main surface 6A of the heat sink body 61, the electronic substrate 5 laminated on the second main surface 6B on the side opposite to the first main surface 6A in the heat sink body 61, and the light irradiation elements 7A and 7B mounted on the first surface 5A, which is the surface of the electronic substrate 5 on the side where the heat sink 6 is provided.

According to the above-described configuration, the heat generated by the light irradiation elements 7A and 7B can be efficiently dissipated by the heat sink 6. Furthermore, both the fin 62 of the heat sink 6 and the light irradiation elements 7A and 7B are provided on the first surface 5A side of the electronic substrate 5. Therefore, for example, compared to a case where both of these extend in mutually different directions, the dimension and the body size of the device can be minimized.

(2) In the light irradiation unit 90 according to a second aspect, the light irradiation elements 7A and 7B are configured to perform light irradiation in the extending direction of the fin 62.

For example, when the light irradiation unit 90 is provided inside the duct 3 of the vehicle air-conditioning device 100, the air blowing circulates around the fin 62. According to the above-described configuration, the heat from the fin 62 can be dissipated by the air blowing, and the light irradiation elements 7A and 7B can be cooled. Furthermore, the light irradiation elements 7A and 7B can stably provide the sterilizing effect for the air blowing itself and other devices exposed inside the duct 3. In particular, light irradiation is performed in the extending direction of the fin 62. In this manner, a time during which the light can be in contact with the air blowing can be secured longer, and sterilization treatment can be more effectively performed.

(3) In the light irradiation unit 90 according to a third aspect, the light irradiation elements 7A and 7B are configured to perform light irradiation toward the side opposite to the side where the heat sink 6 is located.

According to the above-described configuration, for example, when the light irradiation unit 90 is provided inside the duct 3 of the vehicle air-conditioning device 100, the light irradiation elements 7A and 7B can stably provide the sterilizing effect for the air blowing itself and other devices exposed inside the duct 3.

(4) The vehicle air-conditioning device 100 according to a fourth aspect includes the fan 1 that pumps air, the duct 3 in which the air pumped from the fan 1 circulates and to the inner surface (inner wall 3A) of which the light irradiation unit 90 is attached, and the evaporator 4 provided on the downstream side of the duct 3 in the first direction F of the air.

According to the above-described configuration, the light irradiation elements 7A and 7B can stably provide the sterilizing effect for the air blowing itself, the fan 1, and the evaporator 4.

(5) In the vehicle air-conditioning device 100 according to a fifth aspect, the light irradiation elements 7A and 7B are configured to perform light irradiation in the direction facing the fan 1 side and in the direction facing the evaporator 4 side.

According to the above-described configuration, the light irradiation elements 7A and 7B can stably provide the sterilizing effect for the air blowing, the fan 1 exposed inside the duct 3, and the evaporator 4.

(6) The vehicle air-conditioning device 100 according to a sixth aspect further includes the power element 8 disposed on the electronic substrate 5 to control driving of the fan 1, and the harness 9 that supplies power to the power element 8 and the light irradiation element 7A and 7B.

According to the above-described configuration, the power is supplied to the power element 8 and the light irradiation elements 7A and 7B from the single harness 9. Therefore, for example, during maintenance work, the power element 8 and the light irradiation elements 7A and 7B can be brought into a turned-off state at the same time by detaching the harness 9. In this manner, a possibility that a worker may be exposed to harmful light can be reduced.

### Industrial Applicability

According to the present disclosure, it is possible to provide a light irradiation unit that can be installed in a space-saving manner and that minimizes power consumption, and a vehicle air-conditioning device.

### Reference Signs List

100 Vehicle air-conditioning device
90 Light irradiation unit
1 Fan
2 Fan case
3 Duct
3A Inner wall
4 Evaporator
5 Electronic substrate
5A First surface
5B Second surface
6 Heat sink
6A First main surface
6B Second main surface
61 Heat sink body
62 Fin
7A, 7B Light irradiation element
71 Support plate
72 Element body
8 Power element
9 Harness
S Power supply

## Claims

1. A light irradiation unit comprising:
a heat sink having a plate-shaped heat sink body and a fin protruding from a first main surface of the heat sink body;
an electronic substrate laminated on a second main surface on a side opposite to the first main surface in the heat sink body; and
a light irradiation element mounted on a first surface of the electronic substrate, which is a surface on a side where the heat sink is provided.

2. The light irradiation unit according to Claim 1,
wherein the light irradiation element is configured to perform light irradiation in an extending direction of the fin.

3. The light irradiation unit according to Claim 1 or 2,
wherein the light irradiation element is configured to perform light irradiation toward a side opposite to a side where the heat sink is located.

4. A vehicle air-conditioning device comprising:
a fan that pumps air;
a duct in which the air pumped from the fan circulates and to an inner surface of which the light irradiation unit according to any one of Claims 1 to 3 is attached; and
an evaporator provided on a downstream side of the duct in a flowing direction of the air.

5. The vehicle air-conditioning device according to Claim 4,
wherein the light irradiation element is configured to perform light irradiation in each of a direction facing a fan side and a direction facing an evaporator side.

6. The vehicle air-conditioning device according to Claim 4 or 5, further comprising:
a power element disposed on the electronic substrate to control driving of the fan; and
a harness that supplies power to the power element and the light irradiation element.
